Europäisches Patentamt

**European Patent Office**

**Office européen des brevets**

(19)

(11) Veröffentlichungsnummer: **0 025 571**
**B1**

(12) **EUROPÄISCHE PATENTSCHRIFT**

(45) Veröffentlichungstag der Patentschrift: **02.05.84**

(51) Int. Cl.³: **C 12 M 1/04, B 01 F 7/18**

(21) Anmeldenummer: **80105314.1**

(22) Anmeldetag: **05.09.80**

(54) **Verfahren und Vorrichtung zur Verbesserung der Mischgüte flüssiger, insbesondere zäher Medien.**

(30) Priorität: **08.09.79 DE 2936388**

(43) Veröffentlichungstag der Anmeldung:
**25.03.81 Patentblatt 81/12**

(45) Bekanntmachung des Hinweises auf die Patenterteilung:
**02.05.84 Patentblatt 84/18**

(84) Benannte Vertragsstaaten:
**AT CH DE FR GB IT LI NL SE**

(56) Entgegenhaltungen:
AT - A - 175 862
DE - A - 2 753 388
DE - C - 743 984
FR - A - 1 523 664
FR - A - 1 546 174
FR - A - 1 571 582
FR - A - 2 041 779
FR - A - 2 273 062
FR - A - 2 320 984
GB - A - 478 354

(73) Patentinhaber: **HOECHST AKTIENGESELLSCHAFT**
**Postfach 80 03 20**
**D-6230 Frankfurt am Main 80 (DE)**

(72) Erfinder: **Heine, Helmut**
**Kirchgasse 12**
**D-6242 Kronberg/Ts. (DE)**
Erfinder: **Kühn, Karl, Dr.**
**Gimbacher Tann 12**
**D-6233 Kelkheim/Ts. (DE)**
Erfinder: **Sittig, Wolfgang**
**Sulzbacher Weg 2**
**D-6238 Hofheim/Ts. (DE)**

Courier Press, Leamington Spa, England.

## Verfahren und Vorrichtung zur Verbesserung der Mischgüte flüssiger, insbesondere zäher Medien

Zur Durchführung von Reaktionen in der Flüssigphase ist es erforderlich, die benötigten Reaktanden durch Mischen und/oder Rühren an die Stelle des Reaktionsablaufes zu transportieren und die Reaktionsprodukte zu entfernen. Für katalytische Reaktionen sind die Transportwege durch die Verteilung der katalytisch wirksamen Substanzen bestimmt, bei Fermentationen durch die Verteilung der Zellen.

Es ist bekannt, solche Reaktionen in Rührwerksbehältern durchzuführen. Vor allem bei sehr zähen und speziell bei nicht-Newton'schen oder strukturviskosen Reaktionsgemischen entstehen leicht Totzonen in wenig gut gerührten Bereichen, die, durch Entmischungserscheinungen besünstigt, an der allgemeinen Stoffzufuhr und -abfuhr nicht mehr teilhaben. Im Falle von chemischen Reaktionen kann dies zur Bildung von sog. Temperaturnestern führen, da auch die gebildete Reaktionswärme nicht abgeführt wird oder aber es kann zum Erliegen der Reaktion durch Selbsthemmung infolge von Überkonzenntrationen kommen. Bei Fermentationsreaktionen bedingt eine solche Totzonenbildung eine Änderung der erwünschten Stoffwechselreaktionen, ungünstigstenfalls eine irreparable Schädigung der betroffenen Zellen und ihr Absterben. Im Verlauf einer batchweisen Antibiotika-Fermentation von bis zu 300 h Dauer verweilen dabei fast alle Zellen gelegentlich in solchen Zonen, selbst wenn diese nur 2—3% des Reaktorvolumens einnehmen.

Die Rührorgane haben die Aufgabe, das Reaktionssystem zu mischung, eingeblasenes Gas zu zer- und verteilen, die Reaktionswärme über den Reaktormantel und/oder über Kühleinbauten abzuführen und unter Umständen durch Einbringen von Scherkräften Agglomeratbildung über eine bestimmte Größe hinaus zu verhindern. Vor allem zur Verwendung von Fermentationsreaktoren sind bisher solche Rührorgane nach der Qualität des von ihnen bewirkten Stoffübergangs aus dem Gas in die wässrige Phase optimiert worden, weniger nach der Güte ihrer Mischwirkung. Mit steigenden Leistungen der biologischen Zellkulturen sind die Rührerleistung gesteigert und die Reaktorvolumina vergrößert worden; aus Reaktoren, die als homogen gemischt angesehen wurden, entwickelten sich dabei in Zonen gegliederte Apparate, mit Belüftungs-, Entgasungs- und Wärmeaustauschzonen.

Die derzeit üblichen Nährlösungen zeigen nicht-Newton'sches strukturviskoses Verhalten. Die verwendeten Rührer haben meistens drei bis fünf Etagen und ein Rührer-/Tankdurchmesserverhältnis von 0,35 bis 0,65. Damit der Reaktorinhalt sich nicht einfach mit der Rührerdrehzahl mitdreht, sind Stromstörer am Rand des Behälters mit geringem Abstand zur Wand montiert, deren Breite senkrecht zur Rühreranströmung etwa 8 bis 10% des Behälterdurchmessers beträgt. Hinter ihnen bilden sich vor allem bei strukturviskosen Reaktionsgemischen stationäre Wirbel oder gar Totzonen mit den oben beschriebenen Nachteilen aus.

Aus der FR—A—1.571.582 ist bereits eine Rührkesselreaktor für Fermentationszwecke bekannt, der direkt an der Innenwand befestigte Stromstörer besitzt, die schraubenlinienförmig ausgebildet sind und die sich über einen Großteil der Reaktorwand erstrecken. Die Bildung von Totzonen kann dadurch nicht wirksam unterbunden werden. Die Länge der schraublinienförmigen Stromstörer gemäß dieser FR—PS hat außerdem die nachteiligen Folgen, daß sich etwaig darunter gebildete Inhomogenitäten nur langsam ausgleichen und daß sich bei Begasung des Reaktors durch Zusammenlagerung von kleineren Gasblasen Großblasen bilden können.

Auch mit den in der GB—A—478.354 und der FR—A—1.523.644 beschriebenen Stromstörern läßt sich des Entstehen von Totzonen in Wandnähe, insbesondere bei der Rührung von zähen Medien, nicht verhindern. Dieses Problem wird in diesem Druckschriften auch gar nicht angesprochen.

Die Aufgabe der vorliegenden Erfindung bestand nun darin, die Nachteile des Standes der Technik zu vermeiden und insbesondere eine solche Strömungsführung zu bewirken, daß die oben beschriebenen Totzonen nicht auftreten können, die Mischwirkung des Rührorgans und der Wärmeübergang verbessert und die mischungsarme Drehbewegung des im Reaktor befindlichen Reaktionsgemisches verhindert werden.

Die vorliegende Erfindung betrifft daher ein Verfahren zur Verbesserung der Mischgüte flüssiger, zäher Medien in Rührkesselreaktoren mit Stromstören, die eine Ablenkung des in Bewegung versetzten Mediums in Richtung der Reaktorlängsachse bewirken, dadurch gekennzeichnet, daß das von Rührorganen in Bewegung versetzte Medium an einer Stelle oder mehreren Stellen der Reaktorinnenwand in verschiedenen Höhen durch als Stromstörer dienende Ablenkelemente, die in Reaktorwandnähe, jedoch nicht unmittelbar an der Reaktorwand angebracht sind, Richtungsänderung in Richtung der Reaktorlängsachse erfährt, wobei durch diese Richtungsänderung jedoch kein schraubenlinienförmiger Strömungsverlauf bewirkt wird und wobei durch die Umlenkbewegung des strömenden Mediums die Geschwindigkeit in Wandnähe erhöht wird.

Desweiteren hat die vorliegende Erfindung einen Rührkesselreaktor zum Gegenstand der mit den üblichen Rührorganen, sowie mit Stromstörern und gegebenenfalls weiteren Einbauten, Mantelkühlungselementen und Vorrichtungen für die Belüftung versehen ist, wobei diese Stromstörer eine Ablenkung in Richtung der Reaktorlängsachse bewirken, dadurch gekennzeichnet, daß sich als Stromstörer an einer Stelle oder mehreren Stellen des Reaktorumfanges in verschiedenen Höhen Ablenkelemente befinden, die nicht schraubenlinienförmig ausgebildet und in Reaktorwandnähe, jedoch nicht unmittelbar an der Reaktorwand angebracht sind und die eine Richtungsänderung in Richtung der Reaktorlängs achse sowie eine Geschwindigkeitserhöhung in Wandnähe bewirken.

Für das erfindungsgemäße Verfahren kommen grundsätzlich alle flüssigen, zähen Systeme, wie begaste Suspensionen, Emulsionen und/oder Dispersionen in Frage, Insbesondere eignet sich dieses für begaste Fermentationsbrühen.

Die Vorteile des erfindungsgemäßen Verfahrens kommen dabei vor allem bei zähen, insbesondere nicht-Newton'schen, strukturviskosen flüssigen Systemen zum Tragen, deren Viskosität oberhalb von 400 mPa·s, insbesondere bei 1000 bis 5000 mPa·s liegt.

Beispielsweise seien als flüssige, zähe Systeme für die Zwecke der Erfindung genannt: Tyloselösungen, Stärkelösungen, Polymerisationsgemische, Fermentationsbrühen zur Herstellung von Antibiotika wie Penicillin oder Tetracyclin, Chephalosporin, Streptomycin, Xanthanfermentationen udgl.

Die Ablenkung der von der Rührvorrichtung radial und tangential auf die Reaktorwand bewegten Flüssigkeitsströme erfolgt erfindungsgemäß senkrecht zur Anströmrichtung, d.h. in Richtung der Reaktorlängsachse bzw. der Achse der Rührvorrichtung. Zumeist geschieht dies nur in einer Richtung, also nach oben oder nach unten. Unter Umständen kann es jedoch zweckmäßig sein, alternierend nach oben und nach unten abzulenken. Die Größe der Ablenkung ist naturgemäß von dem Anstellwinkel der Einzelelemente der (des) erfindungsgemäßen Stromstörer(s) abhängig; daneben spielen auch noch die Viskosität des flüssigen Mediums und die Rührenergie eine Rolle. Durch die erfindungsgemäße Richtungsänderung wird in Wandnähe des Reaktors eine definierte Vorzugsrichtung der Gemischbewegung hervorgerufen und gleichzeitig die Geschwindigkeit des strömenden Mediums erhöht.

Der erfindungsgemäße Rührkesselreaktor kann grundsätzlich beliebige Form aufweisen; im allgemeinen ist er jedoch als stehender Zylinder ausgebildet.

Der (oder die) entsprechend der erfindungsgemäßen Neuerung darin befindliche(n) Stromstörer besteht (bestehen jeweils) aus einer Anzahl von in Reaktorlängsachse vorzugsweise senkrecht übereinander angeordneter, dünner flächenförmiger Körper, vorzugsweise Platten, die zur Anströmrichtung, d.h. zu einer Ebene senkrecht zur Reaktorlängs (Rührer) achse geneigt sind. Dieser Neigungswinkel ist im allgemeinen um so kleiner, je weniger viskos das Medium und je höher die Anströmgeschwindigkeit ist und beträgt zweckmäßigerweise ±20 bis ±85°, vorzugsweise ±30 bis ±60°. In der Regel ist dieser Winkel für alle Elemente eines Stromstörers gleich. Falls mehrere derartiger Stromstörer im Reaktor vorhanden sind, so können gegebenenfalls die Anstellwinkel von Stromstörer zu Stromstörer verschieden, insbesondere alternierend sein. Im letzteren Fall erfolgt also abwechselnd eine Ablenkung in eine Richtung (nach oben) und die entgegengesetzte Richtung (nach unten).

Die plattenförmigen Körper des erfindungsgemäßen Stromstörers stellen zumeist Rechtecke oder Quadrate dar; sie können dabei eben oder gekrümmt ausgebildet sein. In Sonderfällen können diese plattenförmige Körper auch engmaschige Gitter darstellen, sofern diese den Anströmdruck aushalten. Die Größe dieser Ablenkelemente ist zumeist so, daß der Abstand zur Behälterwand 0,01 bis 0,1 D (D=Reaktordurchmesser), vorzugsweise 0,01 bis 0,05 D beträgt und sie sich dabei vorzugsweise über 8 bis 20% des Behälterdurchmessers in radialer Richtung erstrecken.

Die übereinander angeordneten Ablenkelemente eines Stromstörers sind an einer entsprechenden, in Reaktorlängsrichtung deraufenden Halterung wie Einfach- oder Doppelgestände angebracht, speziell so, daß der Anstellwinkel variabel einstellbar ist. Der Abstand dieser Ablenkelemente untereinander beträgt zweckmäßigerweise 3 bis 30%, vorzugsweise 10 bis 15% des Behälterdurchmessers. Die Zahl der Einzelelemente eines Stromstörers ist abhängig von der Behälterhöhe.

In der Regel werden 2 bis 20 Einzelelemente pro Meter Reaktorhöhe ausreichend sein.

Die Zahl der erfindungsgemäßen Stromstörer liegt zweckmäßigerweise zwischen 1 bis 8; vorzugsweise werden 4 Stromstörer eingesetzt.

In einer weiteren Ausgestaltungsform der Erfindung können die Stromstörer, d.h. die Halterung und die einzelnen Ablenkelemente, zur Aufnahme eines Kühl- oder Heizmittels hohl ausgebildet sein.

Als Rührorgane können erfindungsgemäß grundsätzlich alle hierfür bekannten Vorrichtungen dienen, die eine geeignete Strömungscharakteristik und eine ausreichende Rührleistung ergeben, beispielsweise Impellerrührer, Turbinenrührer, Schrägblatt-Turbinen, Blattrührer, Inter-MIG-Rührer, MIG-Rührer, Frings-Rührer udgl. Derartige, für die Zwecke der Erfindung geeignete Rührorgane sind z.B. beschrieben in dem Artikel von Kipke "Rühren von dünnflüssigen und mittelviskosen Medien", CIT 51 (1979), S. 430—436 oder von Sittig und Heien "Erfahrungen mit großtechnischen eingesetzen Bioreaktoren", CIT 49 (1977), Nr. 8, S. 595—605. Vorzugsweise werden erfindungsgemäß Mehr-Etagen-Flügelrührer oder Mehr-Etagen-Scheibenrührer eingesetzt, wobei der Etagenabstand im allgemeinen 1 bis 3×Rührerdurchmesser beträgt. Die Zahl der Flügel pro Flügelrad liegt im allgemeinen zwischen 3 bis 6 und das Rührer-Reaktordurchmesser-Verhältnis zumeist bei 0,35 bis 0.65.

Daneben enthält der erfindungsgemäße Rührkesselreaktor die üblichen Einbauten, wie Einfüllstutzen, Ventile, Pumpen, Rohrleitungen, Regel, Steuer-, und Meßinstrumente sowie ggf. Anbauten, wie Rückflußkühler.

Falls der erfindungsgemäße Rührkesselreaktor für Fermentierungen eingesetzt wird, sind an ihm auch die hierfür üblichen Vorrichtungen zur Begasung des Reaktionsmediums vorgesehen, d.h. entsprechende Ein- und Ausleitungsrohre sowie üblicherweise eine mit dem Einleitungsrohr verbundene Ringdüse; alternativ kommen hierfür auch eine Einzeldüse oder ein Luftdüsenkranz in Frage. Für den Fall stark endodermer oder exothermer Reaktionen besitzt der Reaktor außerdem einen Doppelmantel für eine entsprechende Mantelkühlung.

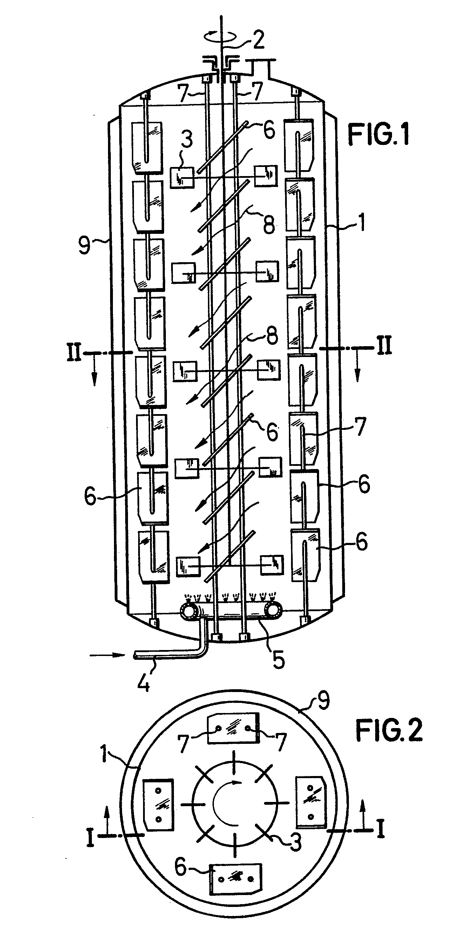

Die anliegenden Figuren erläutern die Wirkungsweise des erfindungsgemäßen Verfahrens und der erfindungsgemäßen Vorrichtung. Figur 1 stellt dabei den Reaktor im Längsschnitt gemäß der Linie I—I der Figur 2 dar, während Figur 2 einen Querschnitt gemäß der Linie II—II von Figur 1 wiedergibt.

In Figur 1 bedeutet (1) den als stehenden Zylinder ausgebildeten Reaktor, (2) den Mehretagen-Flügelrührer mit den Flügeln (3). Das Reaktionsgas wird durch das Einleitungsrohr (4) durch den Düsenring (5) in den Reaktor eingeführt und zusammen mit der fluiden Reaktionssuspension durch den Rührer angesaugt und radial und tangential in Richtung der Reaktorwand bewegt, wo durch den erfindungsgemäßen Stromstörer, bestehend aus den Einzelelementen (6) und der als Doppelstange ausgebildeten Halterung (7) eine Ablenkung nach unten gemäß den Stromlinien (8) erfolgt. Dadurch wird eine definierte Vorzugsrichtung der Gemischbewegung erzeugt und gleichzeitig resultiert eine höhere Geschwindigkeit des Gemisches im Wandbereich, was auch eine Verbesserung des Wärmeübergangs an den Kühlmantel (9) zur Folge hat.

Beispiel 1

In einem 130 1-Rührkessel, der vier der erfindungsgemäßen Stromstörer mit jeweils 8 rechteckigen Ablenkplatten mit einer Größe von jeweils 0,0032 m² enthielt und mit einem Zwei-Etagen-Scheibenrührer (Zahl der Flügel: 6) ausgestattet war, wurde eine 1,3 %ige Tylose-Lösung gemischt und die Mischzeit durch Farbumschlagsmessungen bestimmt. Der Anstellwinkel der Ablenkelemente bei Verwendung der erfindungsgemäßen Stromstörer betrug 45°. Die Ablenkplatten waren durch ein Doppelgestänge gehalten, das am Reaktorboden und Rektorkopf befestigt war; sie erstreckten sich über 10% des Behälterdurchmessers in radialer Richtung und ihr Abstand zur Reaktorwand lag bei 0,01 D.

Bei einem mechanischen Leistungseintrag (Rührerenergie) von 5 kW/m³ betrug die Mischzeit 12 s.

Vergleichsversuch

Bei Verwendung von vier konventionellen senkrecht zur Rühreranströmung angeordneten Stromstörern von 45 mm Breite lag die Mischzeit demgegenüber bei 20 s.

Beispiel 2

Das Beispiel 1 wurde wiederholt, jedoch unter Verwendung eines Blattrührers anstelle des Zwei-Etagen-Scheibenrührers. Die Leistungsaufnahme (Rührenergie) lag bei 1 kW/m³ und die Mischzeit betrug 18 s.

Vergleichsversuch

Bei Verwendung von herkömmlichen Stromstörern unter sonst gleichen Bedingungen wie in Beispiel 2 betrug dagegen die Mischzeit 50 s.

Beispiel 3

Zur Herstellung von Penicillin wurde ein 40 m³-Fermentationskessel benutzt, der vier der erfindungsgemäßen Stromstörer enthielt; die Zahl der rechteckigen Ablenkplatten je Stromstörer betrug 18, der Anstellwinkel lag bei 40°, die Größe der Ablenkplatten war jeweils 0,075 m², sie erstreckten sich über 9% des Behälterdurchmessers in radialer Richtung, der Abstand zur Wand lag bei 0,15 D. Als Rührer wurden drei Turbinenrührer eingesetzt, die Begasung erfolgte mit einer Vorrichtung wie in Figur 1 beschrieben.

In diesen Fermentationskessel wurden 26 m³ Trinkwasser vorgelegt und folgende Rohstoffe eingetragen:

| Sojamehl | 1320 kg |
|---|---|
| Natriumthiosulfat | 250 kg |
| Calciumcarbonat | 230 kg |
| Sojaöl | 165 kg |
| Lactose | 3300 kg |
| Phenoxiessigsäure | 150 kg |

Die Suspension wurde mit Natronlauge auf einen pH-Wert von 6,0 eingestellt und 40 min mit Dampf bei 120° sterilisiert. Nach dem Abkühlen der Nährlösung auf 25°C wurde der Fermentationskessel mit 3000 l gut gewaschener Penicillin-Vorkultur beimpft. Die Fermentierung wurde bei 25°C unter Belüftung mit 0,5 l Luft/l Füllvolumen und einer Rührenergie von 2,5—3 kW/m³ durchgeführt. Zum Entschäumen wurde während der Fermentation mehrmals Sojaöl in Portionen von ca. 5 l zugegeben, wobei die Gesamtmenge 5—1000 l betrug. Die Fermentationszeit betrug 150 Stunden und die Ausbeute 14 950 E/ml.

Vergleichsversuch

Beispiel 3 wurde wiederholt, jedoch in einem Fermentationskessel, der anstelle der vier erfindungsgemäßen Stromstörer konventionelle Stromstörer mit 0,1 D Breite enthielt.

Die Ausbeute lag hier bei 13 500 E/ml.

Beispiel 4

In dem Fermentationskessel gemäß Beispiel 3 wurden 22 m³ Trinkwasser vorgelegt und folgende Rohstoffe eingetragen:

| | |
|---|---|
| Erdnußmehl | 1100 kg |
| Kristallzucker | 1250 kg |
| Ammonsulfat | 143 kg |
| Calciumcarbonat | 310 kg |
| Zitronensäure | 25 kg |
| Schweineschmalz | 65 kg |
| $Na_2HPO_4 \cdot 12\ H_2O$ | 20 kg |
| $MgSO_4 \cdot 7\ H_2O$ | 19 kg |
| $MnSO_4 \cdot 1\ H_2O$ | 2.5 kg |
| $ZnSO_4 \cdot 7\ H_2O$ | 5.0 kg |
| $FeSO_4 \cdot 7\ H_2O$ | 0.310 kg |
| $Al_2(SO_4)_3 \cdot 18\ H_2O$ | 0.310 kg |

Die Nährlösung wurde 30 min bei 120° mit direktem Dampf sterilisiert, gekühlt und bei Erreichen von 28° mit 1500 l gut gewachsener tetracyclin-Vorkultur beimpft. Die Fermentation wird bis zu 12. Stunde bei 28° und dann bei 25°C durchgeführt; die Belüftung erfolgte mit 0,5 l Luft/l Füllvolumen und die Rührenergie betrug 2 kW/m³. Nach ca. 48 Stunden wurde eine Lösung von 750 kg Zucker und 180 kg Ammonsulfat in 2000 l Trinkwasser, die bei 120° 30 min sterilisiert worden war, zugegeben. Eine zweite Nachgabe erfolgte nach etwa 72 Stunden; sie bestand aus einer Lösung von 750 kg Zucker in 2000 l Trinkwasser, die wie vorstehend angegeben, sterilisiert worden war.

Zum Entschäumen wurden während der Fermentation außerdem Lard- und Sajaöl je nach dem Schaumverhalten zugegeben. Das Endvolumen lag schließlich bei 32 m³. Die Fermentation wurde nach 160 Stunden beendet.

Die Ausbeute lag bei 9359 E/ml.

Vergleichsversuch

Beispiel 4 wurde wiederholt, jedoch in einem Fermentationskessel mit 4 konventionellen Stromstörern mit 0,1 D Breite. Die Ausbeute lag hier bei 8300 E/ml.

**Neue Patentansprüche**

1. Verfahren zur Verbesserung der Mischgüte flüssiger, zäher Medien in Rührkesselreaktoren mit Stromstörern, die eine Ablenkung des in Bewegung versetzten Mediums in Richtung der Reaktorlängsachse bewirken, dadurch gekennzeichnet, daß das von Rührorganen in Bewegung versetzte Medium an einer Stelle oder mehreren Stellen der Reaktorinnenwand in verschiedenen Höhen durch als Stromstörer dienende Ablenkelemente, die in Reaktorwandnähe, jedoch nicht unmittelbar an der Reaktorwand angebracht sind, eine Richtungsänderung in Richtung der Reaktorlängsachse erfährt, wobei durch diese Richtungsänderung jedoch kein schraubenlinienförmiger Strömungsverlauf bewirkt wird und wobei durch die Umlenkbewegung des strömenden Mediums die Geschwindigkeit in Wandnähe erhöht wird.

2. Verfahren nach Anspruch 1, dadurch gekennzeichnet, daß die Medien Fermentationsbrühen sind.

3. Rührkesselreaktor, der mit den üblichen Rührorganen sowie mit Stromstörern und gegebenenfalls weiteren Einbauten, Mantelkühlungselementen und Vorrichtungen für die Belüftung versehen ist, wobei diese Stromstörer eine Ablenkung in Richtung der Reaktorlängsachse bewirken, dadurch gekennzeichnet, daß als Stromstörer sich an einer Stelle oder mehreren Stellen des Reaktorumfanges in verschiedenen Höhen Ablenkelemente befinden, die nicht schraubenlinienförmig ausgebildet und in Reaktorwandnähe, jedoch nicht unmittelbar an der Reaktorwand angebracht sind und die eine Richtungsänderung in Richtung der Reaktorlängsachse sowie eine Geschwindigkeitserhöhung in Wandnähe bewirken.

4. Vorrichtung nach Anspruch 3, dadurch gekennzeichnet, daß 1 bis 8 Stromstörer angebracht sind.

5. Vorrichtung nach Anspruch 3 und 4, dadurch gekennzeichnet, daß die Ablenkelemente jedes Stromstörers einen Winkel von 20 bis 85° in Auf- und/oder Abwärtsrichtung zu der Anströmungsrichtung bilden.

6. Vorrichtung nach Anspruch 3 bis 5, dadurch gekennzeichnet, daß die Stromstörer zur Aufnahme eines Heiz- oder Kühlmittels hohl ausgebildet sind.

**Revendications**

1. Procédé pour améliorer, dans un réacteur de type autoclave comportant un dispositif d'agitation, la qualité de mélangeage de milieux fluides ou liquides, visqueux, à l'aide de chicanes qui provoquent une déviation du milieu mis en mouvement dans le sens de l'axe longitudinal de réacteur, procédé caractérisé en ce que le milieu mis en mouvement par les organes d'agitation subit en un ou plusieurs endroits de la paroi interne du réacteur à différentes hauteurs une modification de direction dans le sens de l'axe longitudinal du réacteur, provoquée par des éléments de déviation ou déflecteurs jouant le rôle de chicanes, qui sont disposés au voisinage de la paroi du réacteur mais non pas immédiatement sur cette paroi de réacteur, de sorte que cette modification de direction no provoque cependant pas de comportement hélicoïdal de l'écoulement et de sorte que, du fait du mouvement de déviation du milieu en écoulement, la vitesse de ce milieu soit augmentée au voisinage de la paroi.

2. Procédé selon la revendication 1, caractérisé en ce que les milieux sont des bouillons de fermentation.

3. Réacteur de type autoclave comportant un dispositif d'agitation, et qui est équipé des organes usuels d'agitation ainsi que de chicanes et éventuellement d'autres éléments rapportés, d'éléments de type enveloppes de refroidissement et de dispositifs pour l'aération, ces chicanes provoquant une déviation dans le sens de l'axe longitudinal du réacteur, celui-ci étant caractérisé en ce qu'en un ou plusieurs endroits de la périphérie de ce réacteur et à des hauteurs différentes se trouvent des éléments, à rôle de déviation ou déflecteurs, jouant le rôle de chicanes, qui n'ont pas la forme hélicoïdale et qui sont disposés au voisinage de la paroi du réacteur, mais non immédiatement sur cette paroi et qui provoquent une modification de direction ou déviation dans le sens de l'axe longitudinal du réacteur ainsi qu'une augmentation de la vitesse au voisinage de la paroi.

4. Réacteur selon la revendication 3, caractérisé en ce qu'il comporte un à huit dispositifs de chicanes.

5. Réacteur selon les revendications 3 et 4, caractérisé en ce que les éléments déflecteurs de chaque dispositif de chicanes forment, vers l'amount et/ou vers l'aval par rapport au sens du l'écoulement, un angle de 20 à 85°.

6. Réacteur selon l'une quelconque des revendications 3 à 5 caractérisé en ce que les dispositifs de chicanes sont réalisés creux de manière à pouvoir loger un agent de chauffage ou de refroidissement.

## Claims

1. A process for improving the quality of mixing of liquid viscous media in stirred tank reactors with flow deflector means effecting a change of direction of the approaching media flow in the direction of the longitudinal axis of the reactor which comprises bringing about a change in direction of the medium set in motion by stirring means in the direction of the longitudinal axis of the reactor—this change in direction, however, not causing a helical-shaped flow—at one point or at several points of the inner wall of the reactor at different levels by a plurality of baffle elements arranged close to the reactor wall but not directly thereto, and wherein the speed of flow is increased near the reactor wall by the deflection movement.

2. The process as claimed in claim 1, wherein the media are fermentation broths.

3. Stirred tank reactor equipped with the usual stirring means, with flow deflector means, and optionally with further inserts, jacket cooling elements and aeration means, the flow deflector means effecting a change in direction of the approaching flow in the direction of the longitudinal axis of the reactor, characterized that it contains a plurality of baffle elements acting as flow deflector means and being arranged at one or several points along the periphery of the reactor at different levels close to the reactor wall but not being fixed directly thereto and not having a helical shape, said baffle elements effecting a change in direction of the approaching flow in the direction of the longitudinal axis of the reactor and an increase of the speed of flow near the reactor wall.

4. The reactor as claimed in claim 3, wherein there are arranged from 1 to 8 flow deflector means.

5. The reactor as claimed in claims 3 and 4, wherein the baffle elements of each deflector means form an angle of from 20 to 85°C in upward and/or downward direction to the angle of approaching flow.

6. The reactor as claimed in claims 3 to 5, wherein the deflector means are hollow to take up a heating or cooling medium.

FIG.1

FIG.2